# EUROPEAN PATENT APPLICATION

(11) **EP 1 803 464 A1**
(43) Date of publication of application: **04.07.2007**
(21) Application number: 05783676.9
(22) Date of filing: 16.09.2005
(51) Int. Cl.: A61K 38/00, A61K 35/14, A61K 47/30, A61K 47/36, A61K 47/42, A61P 17/02, A01K 67/027, C12Q 1/02, C12M 1/34

(54) **EXTERNAL PREPARATION FOR TREATING SKIN ULCER**

(30) Priority: 17.09.2004 JP 2004271996; 12.05.2005 JP 2005140301; 19.08.2005 JP 2005239189
(71) Applicant: CellGenTech, Inc., Chiyoda-ku, Tokyo 101-0062 (JP)
(72) Inventor: JIMI, Shiro, Fukuoka-shi, Fukuoka 814-0175 (JP); SUZUMIYA, Junji, Fukuoka-shi, Fukuoka 814-0155 (JP); SATO, Tomohito, Hiroshima-shi, Hiroshima 730-0846 (JP)
(74) Representative: LOUIS, PÖHLAU, LOHRENTZ
(86) International application number: PCT/JP2005/017121
(87) International publication number: WO 2006/030887

(57) **Abstract**

[Problems] To provide a novel external agent for treatment of skin ulcer which has an excellent healing effect on intractable skin ulcer such as bedsore, diabetic skin ulcer and ischemic skin ulcer.

[Means for Solving Problems] The agent is **characterized in that** it comprises a composition containing at least one selected from the group consisting of granulocyte colony stimulating factor (G-CSF), stromal cell-derived factor-1 (SDF-1) and CD41-positive cells, and a hydrophilic high molecular substance.

## Description

### Technical Field

The present invention relates to a novel external agent for treatment of skin ulcer which has an excellent healing effect on intractable skin ulcer such as bedsore (decubitus), diabetic skin ulcer and ischemic skin ulcer.

### Background Art

At present, in advanced countries including Japan, lifestyle-related diseases including diabetes are progressively increasing due to aging of society and changes in lifestyles and the like. When hospitalized patients or bedridden patients suffer from a circulatory disorder due to diabetes or arteriosclerosis, bedsore is formed. Because bedsore is intractable, there has not been an excellent therapeutic method so far, therefore, the development of an effective therapeutic method has been awaited. Bedsore starts to occur at the sacral region or heel region, and the reasons for the occurrence of bedsore are supposed to be a decrease in tissue endurance due to compression, wetting, malnutrition and the like. In particular, skin tissue and subcutaneous tissue become thin and vulnerable in elderly, and a decrease in the tissue healing response is to be a cause of further delaying the bedsore healing. The causes of intractable skin ulcer including bedsore have been believed to be a decrease in the ability of angiogenesis, a disorder of proliferation of fibroblasts, etc., that is, a decrease in the ability to form granulation tissue in an affected area. However, the exact mechanism of granulation tissue formation has not been elucidated yet. Therefore, only a method for maintaining tissue in an affected area, direct spraying of growth factors or the like has been employed as the therapeutic method, and a practical therapeutic method has not been established yet.
Megakaryocytes are progenitor cells of platelets. During the maturation of megakaryocytes, many cell growth factors such as PDGF (platelet-derived growth factor), TGF-β (transforming growth factor-β) and IGF (insulin-like growth factor) and cytokines are synthesized, and finally those are included in platelets. The differentiation process of megakaryocytes in the bone marrow is similar to the other somatic cells, and megakaryocytes have diploid (2N) chromosomes, and in promegakaryocytes in the bone marrow, the ploidy is increased to a tetraploid (4N), and further increased to 8N, 16N and 32N (sometimes to 64N), however, because the cytoplasm does not divide, the cell gradually increases in size. In the cytoplasm in the matured megakaryocyte, the formation of a large number of α granules and demarcation membrane is observed. With regard to the release of platelets, "proplatelets" in the early stage are split and released, which are further split to be matured platelets. The formed platelets promptly adhere to damaged endothelial cells or subendothelial matrix, and degranulate there, whereby many substances included therein are released, which stimulate regeneration of tissue and cells in the vicinity of the damaged site or proliferation of fibroblasts, thereby forming granulation tissue as well as proliferating blood vessels or connective tissue. At present, platelet adhesion is regarded to be the most important rate limiting response for wound tissue repair.
As a chemokine required for differentiation of megakaryocytes in the bone marrow, TPO (thrombopoietin) is known. On the other hand, G-CSF (granulocyte colony-stimulating factor) is a cytokine found to increase neutrophil granulocytes which play a main role in the inflammation such as bacterial infection, and is regarded to be the most important emergency cytokine in the biological defense, and is known to cause mobilization of hematopoietic stem cells. The G-CSF activity is observed in various tissues, and G-CSF is produced in mainly monocyte macrophages, vascular endothelial cells, bone marrow stromal cells and the like and secreted, however, at present, the use of G-CSF as a pharmaceutical product is limited to increase in neutrophils.
Recently, it has been reported that not only local tissue but also bone marrow-derived cells play an important role in tissue repair and/or regeneration. It has been reported that in the case where tissue is damaged, bone marrow-derived fibrocytes present in the blood are mobilized into the damaged site and differentiate into myofibroblasts, however, the details have not been elucidated yet. Recently, it has been reported that a wound healing effect can be obtained by intraperitoneal injection of G-CSF (Non-patent document 1). Further, in Patent document 1, a method of accelerating wound healing by local application or non-oral administration of G-CSF or GM-CSF (granulocyte-macrophage colony-stimulating factor) has been proposed. However, in Patent document 1, there is no data showing the effects associated with G-CSF, and there is only data of GM-CSF. Since G-CSF and GM-CSF have completely different biological functions, even a person skilled in the art cannot speculate the effects of G-CSF from the data of GM-CSF. Therefore, the effects of G-CSF by directly applying it to an affected area as an external agent or subcutaneously injecting it to an affected area still remained unknown. Further, it has been reported that SDF-1 (stromal cell-derived factor-1, another name: CXCL12), which belongs to the α chemokine (CXC) family and is a physiological ligand for CXCR4 (T-tropic HIV-1 coreceptor), is important for megakaryocytes to migrate (home) to the niche beneath the endothelial cells to release platelets in the bone marrow, and that SDF-1 is involved in accumulation of leukocytes in an affected area from the blood in the process of wound healing (Non-patent document 2 and Non-patent document 3). However, the effects of local application of SDF-1 are still unknown.
Patent document 1: JP-T-5-506673
Non-patent document 1: Eroglu E, et al., Effects of granulocyte-colony stimulating factor on wound healing in a mouse model of burn trauma. Tohoku J. Med. 204, 11-16 (2004)
Non-patent document 2: Heissing B et al., Recruitment of stem and progenitor cells from bone marrow niches MMP-9 mediated release of kit-ligand. Cell 109, 625-637 (2002)
Non-patent document 3: Daniel J Ceradini et al., Progenitor cell trafficking is regulated by hypoxic gradients through HIF-1 induction of SDF-1. Nature Medicine 10, 858-864 (2004)

### Disclosure of the Invention

### Problems that the Invention is to Solve

Thus, an object of the present invention is to provide a novel external agent for treatment of skin ulcer which has an excellent healing effect on intractable skin ulcer such as bedsore, diabetic skin ulcer and ischemic skin ulcer.

### Means for Solving the Problems

The present inventors have made intensive studies targeting intractable skin ulcer, which is often observed in the patients with diabetes, and aiming at elucidating the mechanism of skin regeneration and establishing a therapeutic method for the disease. At present, as a treatment method for ischemic tissue, regeneration medicine aiming at accelerating a wound healing effect in which cells are isolated and collected, the differentiation thereof into a specific type of cells by a growth factor or the like under culture conditions is induced, and then, the resulting cells are injected into the wound tissue has also been examined, although the role of the stem cells (progenitor cells) in the wound healing process has remained uncertain. However, such a method cannot be a practical therapeutic method for intractable skin ulcer. At the beginning, the present inventors studied the mechanism of the wound healing process using healthy normal mice. As a result, they found that in the phenomenon associated with skin tissue repair, there are shrinkage of wound area and formation of granulation tissue, and both events interact with each other in corporation. Further, they elucidated that in shrinkage of wound area, bone marrow-derived cells are liable for about 60% and peripheral tissue cells are liable for the rest, i.e., about 40%. In the formation of granulation tissue, bone marrow-derived myofibroblasts are the most important, and when the bone marrow does not function, the formation of granulation tissue does not occur at all. These basic findings had not been elucidated at all before. Moreover, on the basis of these basic findings, it was found that organic interaction of G-CSF, which is capable of mobilizing stem cells and megakaryocytes (incidentally, the action of mobilizing megakaryocytes by G-CSF is also a new finding discovered this time by the present inventors), CXCR4, which is carried by bone marrow-derived stem cells, and a chemokine such as SDF-1, which is a ligand for CXCR4, in the wound area is extremely important for the repair of wound.

An external agent for treatment of skin ulcer according to the present invention which has been made based on the above-mentioned findings is characterized in that, as described in claim 1, it comprises a composition containing at least one selected from the group consisting of G-CSF, SDF-1 and CD41-positive cells, and a hydrophilic high molecular substance.
Further, an external agent for treatment of skin ulcer as described in claim 2 is characterized in that, in the external agent for treatment of skin ulcer described in claim 1, the hydrophilic high molecular substance is at least one selected from the group consisting of collagen, alginic acid and salts thereof.
Further, an agent for local application for treatment of intractable skin ulcer of the present invention is characterized in that, as described in claim 3, it contains G-CSF as an active ingredient.
Further, an agent for treatment of skin ulcer of the present invention is characterized in that, as described in claim 4, it contains SDF-1 as an active ingredient.
Further, an agent for treatment of skin ulcer of the present invention is characterized in that, as described in claim 5, it contains CD41-positive cells as an active ingredient.
Further, an animal model of skin ulcer of the present invention is characterized in that, as described in claim 6, it is produced by administering an anti-tumor drug to a non-human animal and 1 to 3 days thereafter, excising the dorsal skin of the animal.
Further, a device for evaluating new tissue generation of the present invention is characterized in that, as described in claim 7, it comprises an extracellular matrix sheet having a predetermined thickness, a cell-permeable membrane and a cell-impermeable membrane, in which the cell-permeable membrane is disposed on one face of the extracellular matrix sheet and the cell-impermeable membrane is disposed on the other face of the extracellular matrix sheet, and that a biological tissue is brought into contact with the cell-permeable membrane as the facing surface, and whether or not new tissue generation occurs in the matrix components by cell migration from the biological tissue is monitored, whereby the degree of new tissue generation can be evaluated.

### Effect of the Invention

According to the present invention, a novel external agent for treatment of skin ulcer which has an excellent healing effect on intractable skin ulcer such as bedsore, diabetic skin ulcer and ischemic skin ulcer is provided.

### Brief Description of the Drawings

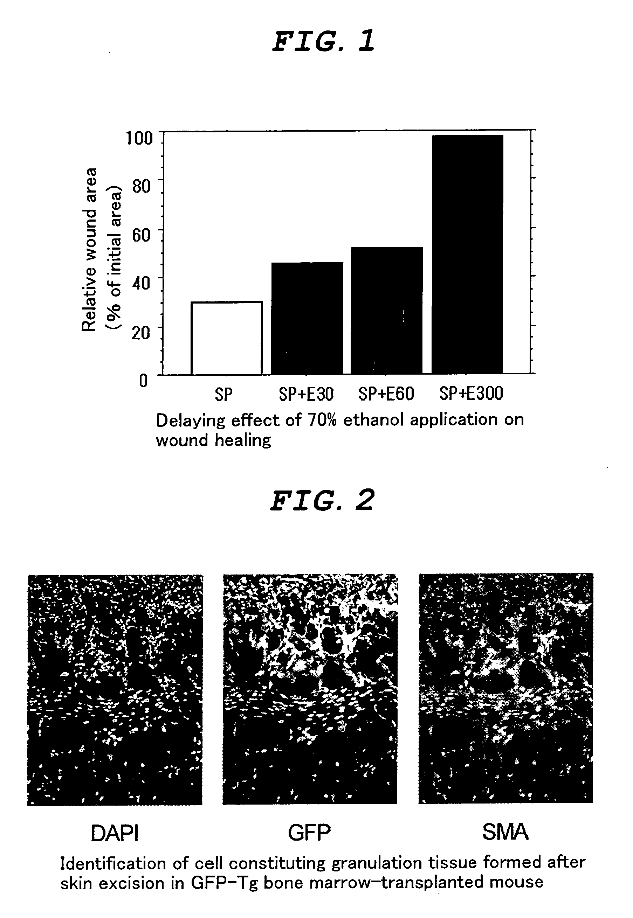

Fig. 1: Delaying effect of dorsal skin excision and ethanol exposure on wound healing: Shrinkage of wound area was reduced according to increase in the time of ethanol exposure (skin excision alone (SP), 30 sec ethanol exposure (E30), 60 sec ethanol exposure (E60) and 300 sec ethanol exposure (E300)).
Fig. 2: Bone marrow-derived GFP cells and α-smooth muscle actin (SMA) expression in granulation tissue: Most of the infiltrating and proliferating cells in granulation tissue were GFP-positive cells having bone marrow-derived nucleus (DAPI), and spindle-shaped cells constituting granulation tissue express α-SMA, therefore, they could be confirmed to be myofibroblasts.

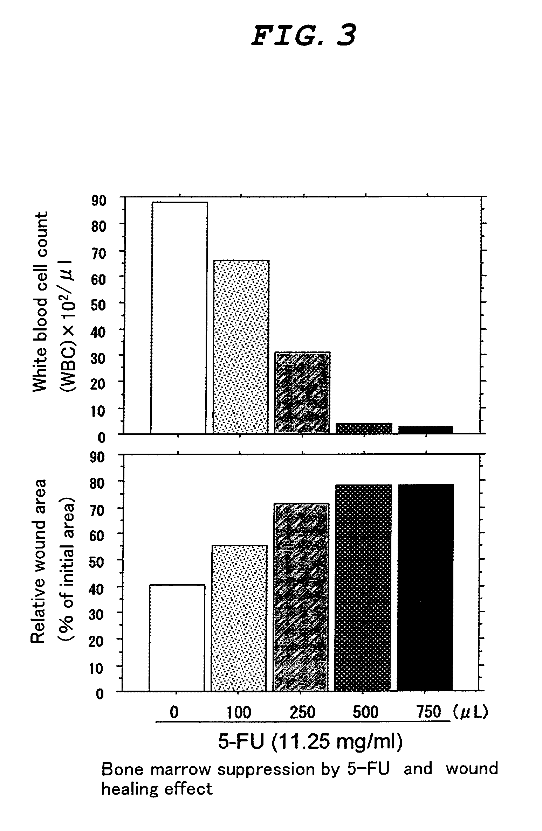

Fig. 3: Bone marrow suppression by 5-FU and wound healing effect: After 5-FU was intraperitoneally administered to C57BL/6 mice at various concentrations, full thickness dorsal skin tissue with a size of 2 x 1.5 cm was excised, and exposure of 70% ethanol for 60 sec was performed. The correlation between the white blood cell count and the relative wound area after 7 days was examined. Both showed a clear negative correlation.

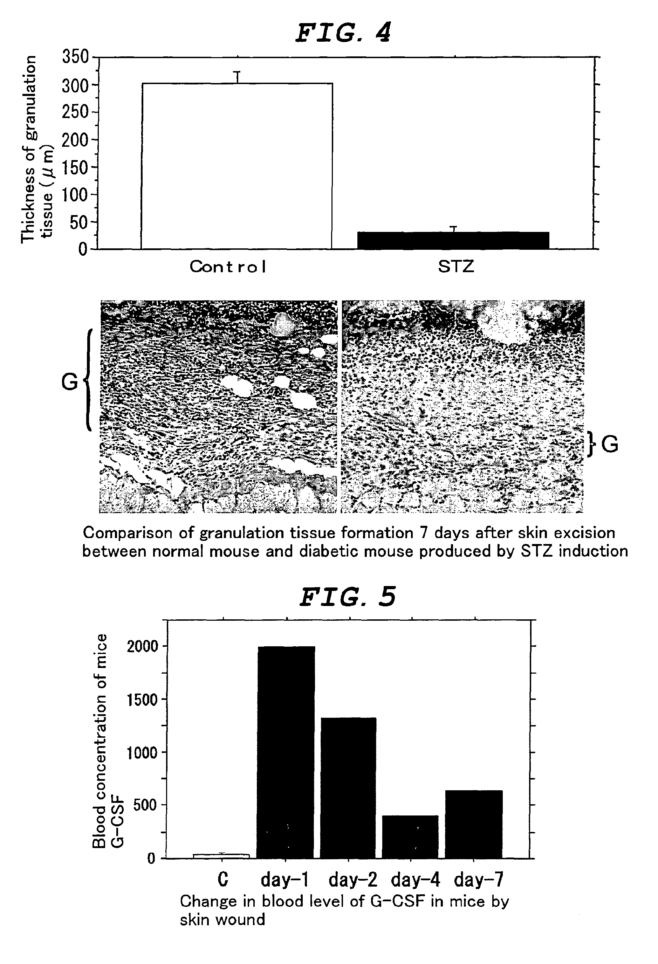

Fig. 4: The thickness of granulation tissue (G) in the healed tissue and the images of the tissue 7 days after performing dorsal skin excision and ethanol exposure in a model of intractable diabetic skin ulcer (right) produced by STZ induction and an ulcer model of delayed skin wound healing (left): In the former, an ability to form granulation tissue was suppressed about 10 times more strongly and infiltration of spindle-shaped cells associated with granulation tissue formation was clearly small, therefore, this model could be used as a model of intractable skin ulcer caused by diabetes.
Fig. 5: Change in the blood level of endogenous G-CSF in mice after dorsal skin excision and ethanol exposure: Endogenous G-CSF level increased drastically 1 day after skin excision and ethanol exposure, but the level gradually decreased thereafter.

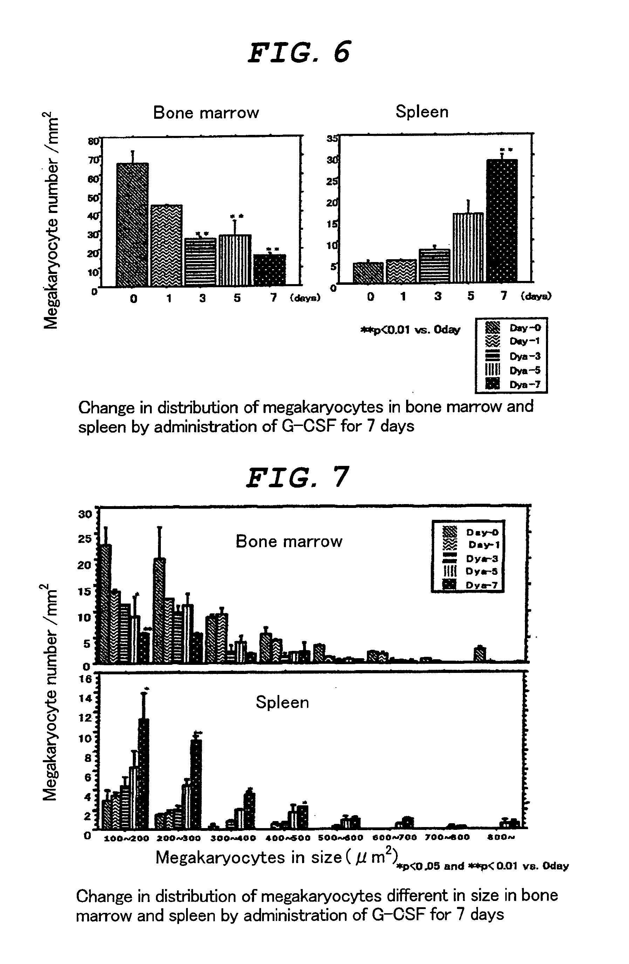

Fig. 6: Change in the megakaryocyte number in the bone marrow and spleen by continuous subcutaneous administration of G-CSF for 7 days: The megakaryocyte number in the bone marrow decreased in accordance with progression of time of study. On the contrary, the megakaryocytes in the spleen increased in accordance with progression of time of study.
Fig. 7: Change in the distribution of megakaryocytes different in size in the bone marrow and spleen by continuous subcutaneous administration of G-CSF for 7 days: Percentage of change in the number of AChE-positive megakaryocytes were large in smaller sized megakaryocytes in both the bone marrow and spleen. The number in smaller sized megakaryocytes decreased in the bone marrow, but increased in the spleen.

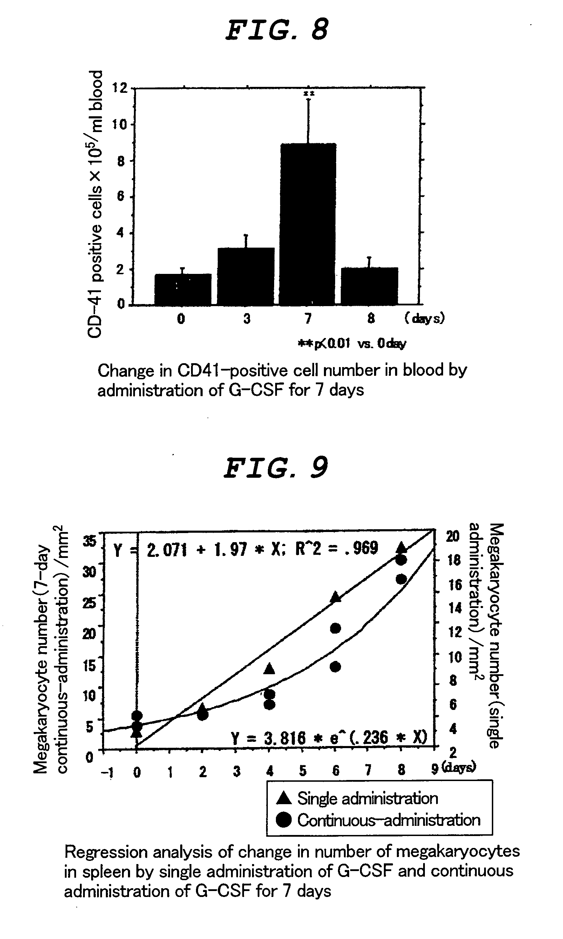

Fig. 8: Change in the CD41-positive cell number in the blood by continuous subcutaneous administration of G-CSF for 7 days: The CD41-positive cells in the peripheral blood gradually increased during G-CSF administration, and after 7 days, the cell number reached about 5 times greater than the level before administration. However, when the administration was discontinued (after 8 days), the cell number decreased to the cell number level before administration.
Fig. 9: Regression analysis of change in the number of megakaryocytes in the spleen by single subcutaneous administration of G-CSF and continuous subcutaneous administration of G-CSF for 7 days: In the single administration of G-CSF, the change in the number of megakaryocytes in the spleen was expressed by a linear regression, and in the continuous administration of G-CSF for 7 days, it was expressed by a quadratic regression, and the effect of G-CSF on releasing megakaryocytes from the bone marrow was shown to be direct.

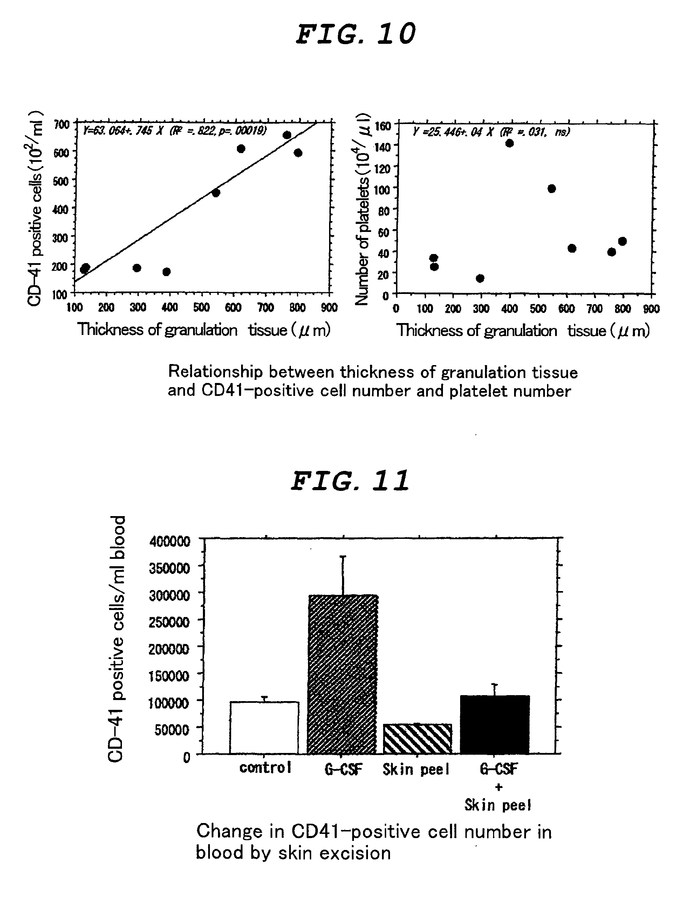

Fig. 10: Relationship between the thickness of granulation tissue and the CD41-positive cell number in the blood and the platelet number in the blood: The thickness of granulation tissue and the CD41-positive cell number in the blood showed a significant positive correlation, however, there was no correlation between the thickness of granulation tissue and the number of platelets produced from megakaryocytes.
Fig. 11: Change in the CD41-positive cell number in the blood by skin excision and ethanol exposure: CD41-positive cells in the blood significantly increased by G-CSF administration were decreased by skin excision and ethanol exposure.

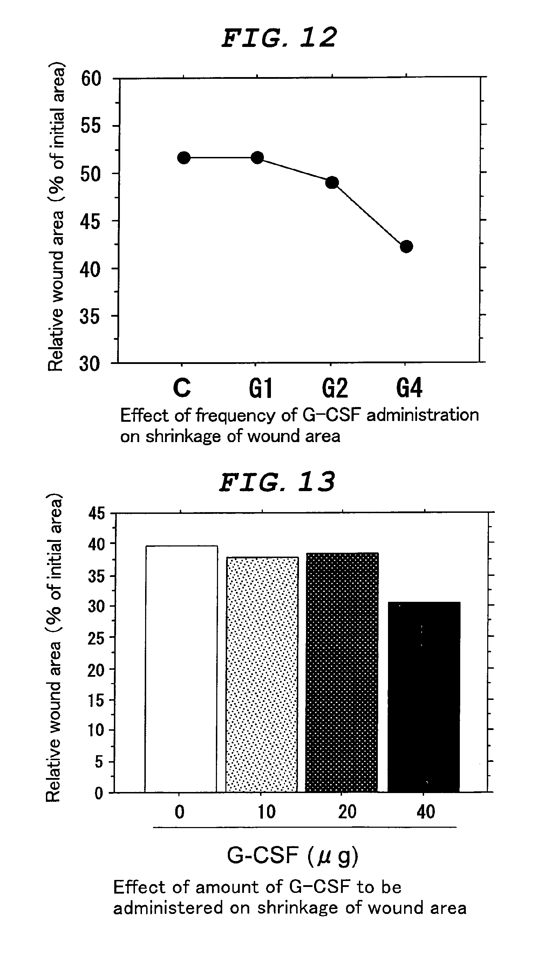

Fig. 12: Effect of frequency of G-CSF subcutaneous administration on shrinkage of wound area: By increasing the frequency of G-CSF administration, the wound area gradually shrank and the healing effect was promoted.
Fig. 13: Effect of amount of G-CSF to be subcutaneously administered on shrinkage of wound area: By increasing the administration amount of G-CSF, a wound area gradually shrank and the healing effect was promoted.

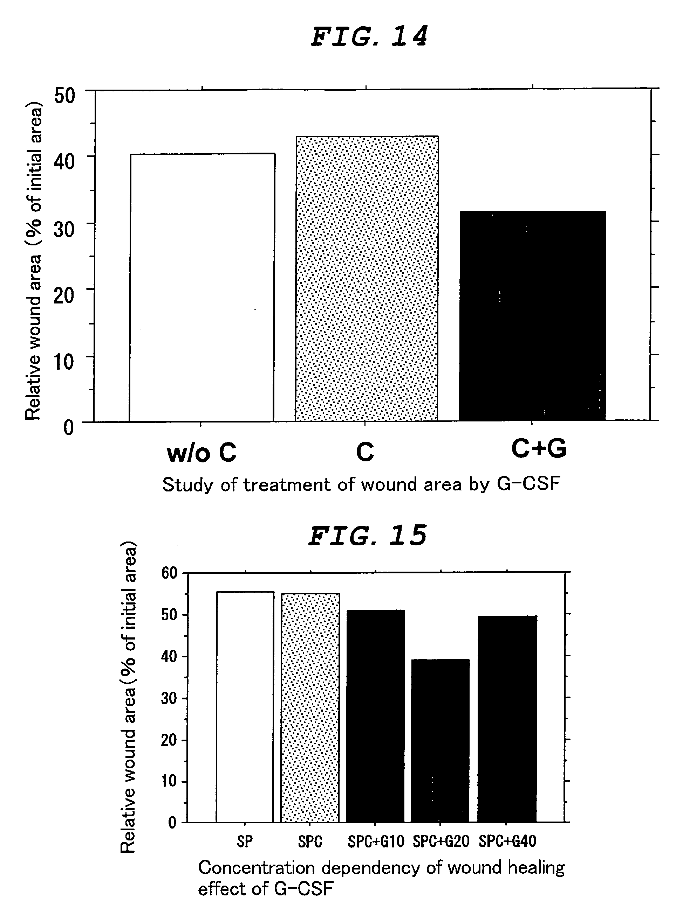

Fig. 14: Wound healing effect in the case where G-CSF was applied by using atherocollagen as a substrate: A wound healing effect was observed by applying G-CSF to a wound area.
Fig. 15: Concentration-dependent wound healing effect in the case where G-CSF was applied by using atherocollagen as a substrate: A relative wound area decreased until the concentration of G-CSF reached 20 µg/200 µl, and the healing effect decreased in the case where the concentration of G-CSF was 40 µg/200 µl although the healing effect was observed.

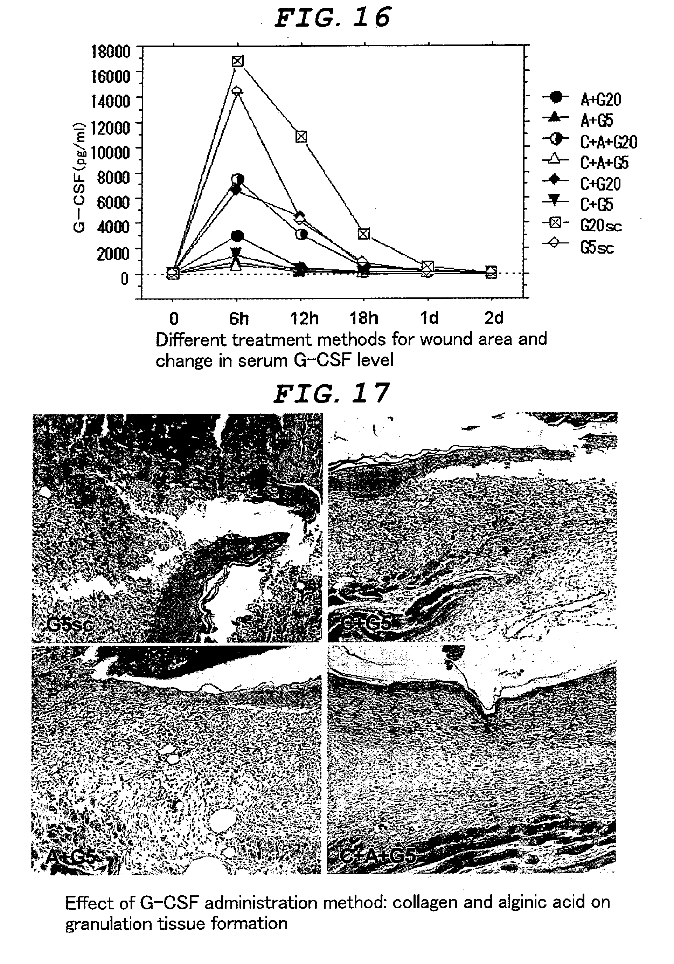

Fig. 16: Relationship between the different administration method of G-CSF and the degree of leakage of G-CSF into blood in ulcer models of delayed skin wound healing: A peak could be observed 6 hours after administration of G-CSF in any method, however, the leakage amount into blood was the largest in the case of subcutaneous administration, and when G-CSF was applied using atherocollagen or alginic acid as a substrate, the leakage amount was small, therefore, this method had a high sustained release effect.

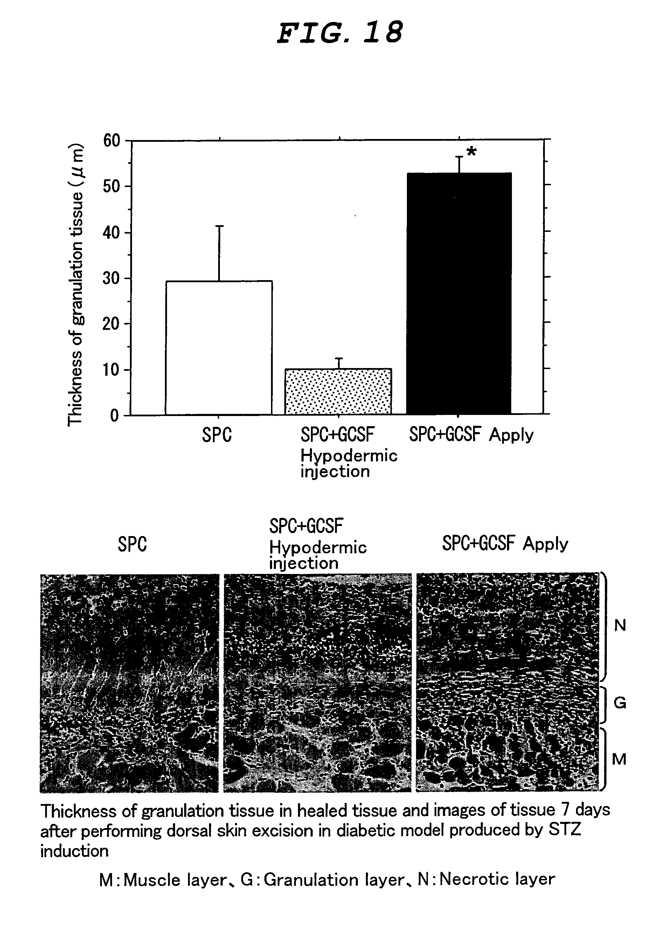

Fig. 17: Effect on granulation tissue formation in the case where G-CSF was applied by using atherocollagen and/or alginic acid as a substrate in ulcer models of delayed skin wound healing: No significant difference in the thickness of granulation tissue was found among the groups. However, particularly when both atherocollagen and alginic acid were used as a substrate, while infiltration of neutrophils to infiltrate was suppressed, neovascular and fibroblast number increased and their arrangement was proper, therefore, it was considered that these matrix components provide a source for a preferable scaffold for the formation of new granulation tissue.
Fig. 18: Therapeutic effect of G-CSF external agent on skin ulcer using models of intractable diabetic skin ulcer (thickness of formed granulation tissue and image of the tissue): In comparison with G-CSF non-administration group (SPC) in which the granulation tissue formation was exacerbated in the diabetic pathologic conditions, in G-CSF application group, thick granulation tissue was formed, and the proliferation of fibroblasts was also favorable, and a healing effect was promoted.

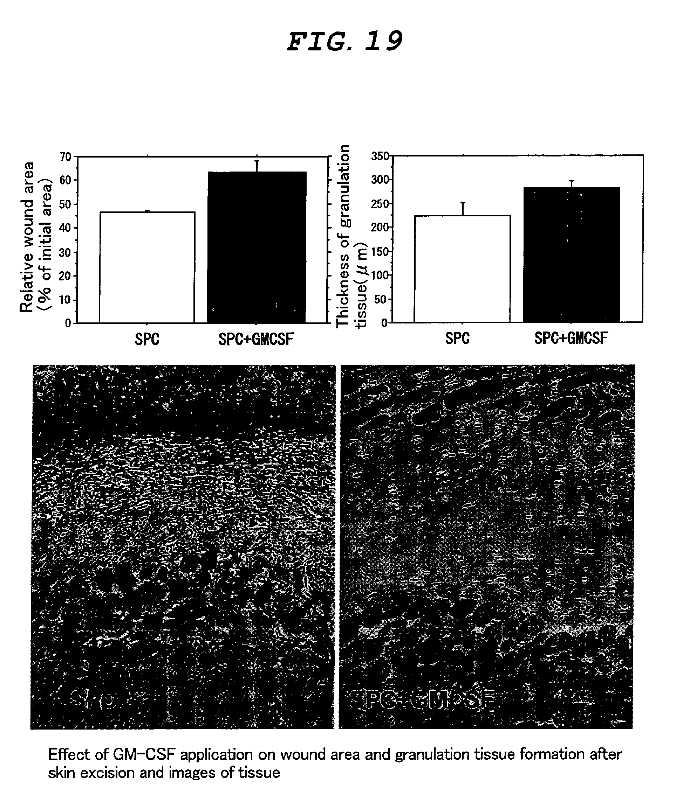

Fig. 19: Effect of GM-CSF on wound healing using ulcer models of delayed skin wound healing (Comparative example): Although the granulation tissue formation accelerating effect of GM-CSF on skin wound was observed to some extent, a negative effect on shrinkage of wound area was observed. Further, by the administration of GM-CSF, swelling of wound area was macroscopically noted and an edematous change was histologically observed.

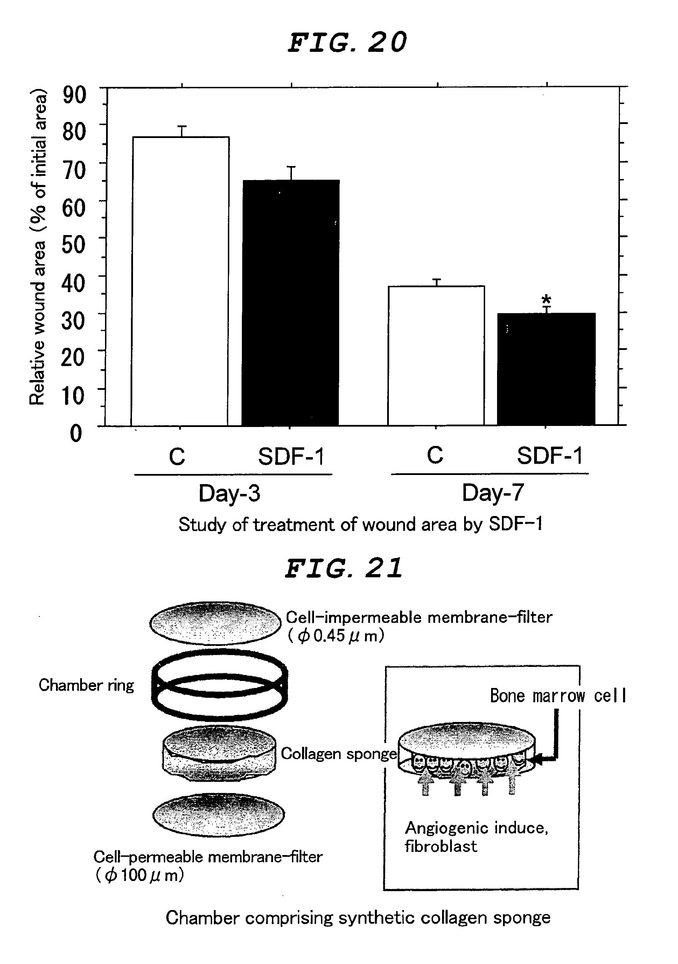

Fig. 20: Therapeutic effect of SDF-1 application in ulcer models of delayed skin wound healing: A wound area significantly shrank by applying SDF-1 to the wound area.
Fig. 21: A block diagram of a device for evaluating new tissue generation.

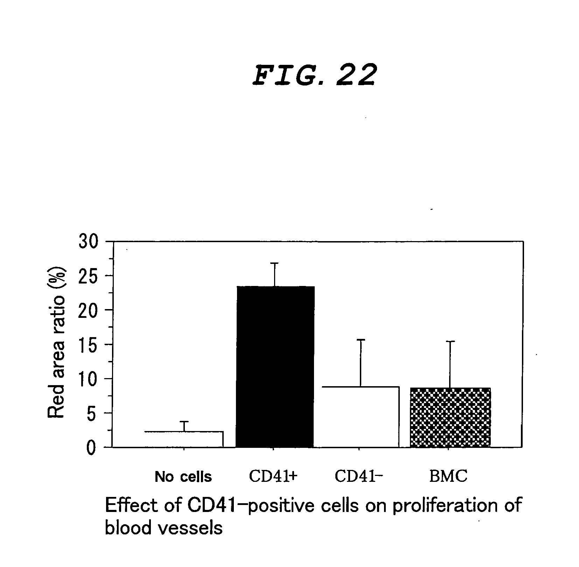

Fig. 22: Effect of CD41-positive cells on proliferation of blood vessels: In comparison with the case where CD41-positive cells were not injected into a synthetic collagen sponge (no cells), in the case where CD41-positive cells were injected into a synthetic collagen sponge, the effect on proliferation of blood vessels was especially promoted.

### Best Mode for Carrying Out the Invention

G-CSF to be an active ingredient of an external agent for treatment of skin ulcer of the present invention is not limited to a natural material derived from a human and having a known amino acid sequence, and may be a substance produced by a genetic engineering technique, or an analogue in which one or more amino acids have been substituted, deleted, added or inserted in the amino acid sequence, as long as it can be recognized as G-CSF by a person skilled in the art, for example, it has an action of differentiating and proliferating granulocytes. The main action of G-CSF in the wound healing is to help mobilization of the whole tissue repair cells such as neutrophils, fibrocytes and megakaryocytes from the bone marrow. This action can be expected to be reinforced by using another vascular endothelial cell growth factor, for example, fibroblast growth factor (FGF) or the like in combination, and can further amplify the ability to form granulation tissue. Therefore, the administration of G-CSF increases a plurality of bone marrow-derived cells required for tissue repair in the blood for intractable skin ulcer in which the ability to heal wound has been lowered. As a result, the maximum potential healing ability in wound area is exhibited, whereby a basic response for healing intractable skin ulcer is accelerated. Further, as an effect of local application (subcutaneous injection and external application) of G-CSF to an area of skin ulcer, a wound healing accelerating effect by acceleration of differentiation of bone marrow-derived cells with low differentiation such as stem cells infiltrated into the vicinity of the area of skin ulcer is also possible. SDF-1 is not limited to a natural material derived from a human and having a known amino acid sequence, and may be a substance produced by a genetic engineering technique, or an analogue in which one or more amino acids have been substituted, deleted, added or inserted in the amino acid sequence, as long as it can be recognized as SDF-1 by a person skilled in the art, for example, it has an action as a physiological ligand for CXCR4. Further, it may be a peptide or a low-molecular synthetic compound having an action as a physiological ligand for CXCR4. It is thought that bone marrow-derived tissue repair cells having CXCR4 recognize SDF-1 expressed in a wound area as a target protein and infiltrate it. Therefore, when an increase in the tissue repair cells from the bone marrow in the blood can be confirmed, by increasing the concentration of SDF-1 in the wound area, the tissue repair cells can be selectively mobilized into the wound area. SDF-1 can be used for induction of mobilization of granulation tissue forming cells which are insufficient in a lesion of intractable skin ulcer and for induction of granulation tissue formation by the mobilization. The action of SDF-1 can be expected to be reinforced by using another vascular endothelial cell growth factor, for example, FGF or the like in combination. As the CD41-positive cell, megakaryocytes and platelets in which surface expression of CD41 is observed can be exemplified. For example, the CD41-positive cells collected from autologous bone marrow secrete a large amount of various types of growth factors carried in the cells without causing rejection response and accelerate the proliferation of fibroblasts or vascular endothelial cells required for granulation tissue formation by administering to a wound area. Therefore, the cells accelerate the granulation tissue formation in the lesion under an optimal condition and exhibit a favorable wound healing accelerating effect.

Examples of the hydrophilic high molecular substance include collagen (aterocollagen which is solubilized by a protease and free from immunogenicity is preferable), alginic acid, a salt thereof (a sodium salt, etc.) and the like. These substances have a high moisturizing effect, and collagen has a sustained release effect of a macromolecule such as a protein, and alginic acid has an effect of accelerating tissue infiltration or intracellular intake of a macromolecule, therefore, they function advantageously in the course of wound healing process.

With regard to the external agent for treatment of skin ulcer of the present invention, for example, a composition obtained by dissolving or dispersing at least one active ingredient selected from the group consisting of G-CSF, SDF-1 and CD41-positive cells in a hydrophilic high molecular substance or a dilution solution thereof using a solvent (preferable examples of the composition include a composition obtained by dissolving G-CSF and/or SDF-1 at a content of 0.001 mg to 10 g/100 ml in a hydrophilic high molecular substance solution prepared by dissolving a hydrophilic high molecular substance at a concentration of 0,01 mg to 10 g/100 ml in a solvent such as purified water, a physiological saline solution, a phosphate buffer solution or a hydrochloric acid solution and a composition obtained by dispersing CD41-positive cells at a cell density of 1 x 10⁵ to 1 x 10¹⁰ cells/100 ml) may be used as an external preparation as such, or such a composition may be used by appropriately formulating it into an ointment, a gel, a cream, a lotion or the like using a well-known base material or solvent as needed. By any method, for example, by applying or spraying this composition to a wound area one to several times a day or once per one day or two days for one week to one month at a dose of 0.1 to 500 µg/cm² in terms of the active ingredient, an excellent therapeutic effect can be expected. Further, this composition may be prepared as a cataplasm by applying this composition to a well-known base material sheet (lint cloth, non-woven cloth or the like), or as a wound dressing by applying this composition to a dressing material composed of polyurethane film for wound dressing and used by applying the resulting product to a wound area. In this case, for example, the wound dressing may be applied to a wound area for one week to one month while replacing it once per one day or two days such that the active ingredient is administered to the wound area at a dose of 0.1 to 500 µg/cm². In addition, a quick-drying spray which should be prepared immediately before use may be made from a freeze-dry product of G-CSF and/or SDF-1 (for example, containing 280 µg of G-CSF and/or SDF-1 in one vial) and a solution of the product (for example, containing 0.3 g of collagen and/or 5 g of alginic acid in 100 ml of purified water), and sprayed in an amount of, for example, 20 µg in terms of G-CSF and/or SDF-1 per 3 cm² of wound area as a standard at one time. It goes without saying that various components such as a well-known stabilizer, thickener, solubilizer, preservative, extending agent, tonicity agent, disinfectant, antiseptic and gelling agent can be added upon formulation of various preparations.

Incidentally, G-CSF, SDF-1 or CD41-positive cells can be used as an active ingredient of the agent for treatment of skin ulcer also in the form of an injection prepared by a well-known method through subcutaneous injection or intravenous injection.

### Examples

Hereinafter, the present invention will be described in detail with reference to Examples. However, it shall be noted that the present invention should not be construed to be limited to the following description.

### (1) Production of ulcer model of delayed skin wound healing

By using a C57BL/6 mouse, an ulcer model of delayed skin wound healing was produced. When full thickness dorsal skin tissue with a size of 2 x 1.5 cm is excised under anesthesia, there is almost no bleeding, and on the surface of the wound area, the fascia is exposed. Immediately thereafter, exposure of 70% ethanol is carried out to the wound area, whereby it becomes possible to induce coagulative necrotic changes in tissue around the wound area. More specifically, the ulcer model of delayed skin wound healing was produced by the following method. Full thickness dorsal skin tissue of a C57BL/6 mouse with a size of 2 x 1.5 cm was excised (SP), the wound area was completely covered with cotton which had been sufficiently soaked in 70% ethanol, whereby ethanol exposure for 30 sec (E30), 60 sec (E60), 300 sec (E300) and 600 sec was carried out. Then, the wound area was air-dried, and the wound area was measured (day 0). 7 days after completion of the study, the wound area was measured, which was compared with the wound area on day 0, and a change in the wound area was examined (the ratio of a change was specified to be a relative wound area and expressed as % of initial area: hereinafter the same shall apply). As a result, the shrinkage of wound area was reduced as the time of ethanol exposure was increased, and the shrinkage hardly occurred by the 300 sec exposure (Fig. 1). Incidentally, the mouse subjected to 600 sec ethanol exposure died 3 days after the study due to deterioration of systemic symptoms. In the case where the time of ethanol exposure was short such as 30 sec exposure or 60 sec exposure, the healing was delayed because the tissue around the wound area directly received degenerative damage due to ethanol, however, granulation tissue was formed in the wound area in one week after the ethanol exposure, and the wound area was covered with epidermal tissue in about 3 weeks and the healing was completed. The 60 sec exposure was to be a factor of delaying shrinkage of wound area, but there was no systemic symptom and its effect was sufficient, therefore, in this Example, this mouse was adopted as an ulcer model of delayed skin wound healing.
A GFP-Tg (enhanced green fluorescence protein expressing mouse) bone marrow-transplanted C57BL/6 mouse was used, skin excision and ethanol exposure were carried out in accordance with the method for producing the ulcer model of delayed skin wound healing, and granulation tissue was observed. As a result, most of the cells constituting the granulation tissue were GFP-positive cells. When the expression of α-smooth muscle actin (α-SMA) in infiltrating cells in this granulation tissue was examined immunohistochemically using the same section, spindle-shaped cells expressing α-SMA (SMA) were observed in the bone marrow-derived GFP-positive cells (GFP), and differentiation into myofibroblasts was shown (Fig. 2). From the above findings, it was considered that bone marrow-derived cells contribute to the healing of skin wound area, that is, granulation tissue formation.

### (2) Production of model of intractable skin ulcer by bone marrow suppression

It was shown that most of the cells constituting granulation tissue formed in the skin wound area are derived from bone marrow, and the cells are locally differentiated into myofibroblasts and repair the tissue. Next, bone marrow suppression was carried out by administering 5-fluorouracil (5-FU), which is a fluorouracil anti-tumor drug, and an ability to form granulation tissue was examined. 750 µl of 5-FU at a concentration of 11.25 mg/ml (sterile purified water was used as the solvent, hereinafter the same shall apply) was intraperitoneally administered to a C57BL/6 mouse. On the following day, full thickness dorsal skin tissue of the mouse with a size of 2 x 1.5 cm was excised, exposure of 70% ethanol was carried out for 60 sec, and granulation tissue formed 7 days thereafter was compared with that of a normal mouse. As a result, the formation of granulation tissue in the mouse subjected to bone marrow suppression by 5-FU was strongly suppressed and also few infiltrating cells were observed. In the same way, 5-FU was administered to a GFP-Tg bone marrow-transplanted C57BL/6 mouse, skin excision and ethanol exposure were carried out, and granulation tissue was observed. As a result, the infiltration of GFP-containing cells into granulation tissue dramatically decreased.
To C57BL/6 mice, 5-FU at a concentration of 11.25 mg/ml was intraperitoneally administered in an amount of 100, 250, 500 and 750 µl, respectively, and on the following day, dorsal skin excision and ethanol exposure were carried out. When the relative wound area and the white blood cell count 7 days after skin excision and ethanol exposure were compared with those of a mice subjected to skin excision and ethanol exposure without 5-FU administration, a decrease in the white blood cell count was observed depending on the concentration of 5-FU. On the other hand, the degree of the decrease in the relative wound area decreased depending on the concentration of 5-FU. From these results, it was found that the bone marrow cells were closely related to the skin wound healing (Fig. 3).
From the above results, the poor formation of granulation tissue after skin wound due to 5-FU administration is considered as one of the models of intractable skin ulcer due to bone marrow dysfunction. Moreover, it was found that when an anti-tumor drug such as a fluorouracil anti-tumor drug having an action of causing dysfunction of bone marrow formation or hematopoiesis is non-orally administered (e.g., intraperitoneal or intravenous administration) to a non-human animal typified by an animal belonging to the Rodent family such as a rat or a mouse at a dose of 1 to 1000 mg/kg, and the dorsal skin was excised 1 to 3 days thereafter, intractable skin ulcer can be formed in the skin excised area.

### (3) Production of model of intractable diabetic skin ulcer by streptozotocin (STZ) induction

A model of diabetes with hyperglycemia was produced by intraperitoneally administering STZ (4 mg/20 g of body weight, as a solvent, a 0.2 M citrate buffer solution (pH 4.8) was used) to a C57BL/6 mouse. By the STZ administration, bone marrow formation was transiently suppressed, however, it was recovered after 2 weeks. 3 weeks after STZ administration, full thickness dorsal skin tissue of the mouse with a size of 2 x 1.5 cm was excised, exposure of 70% ethanol was carried out for 60 sec, and a relative wound area and an ability to form granulation tissue 7 days thereafter were compared with those of an ulcer model of delayed skin wound healing using a normal mouse. As a result, the relative wound area was about 80% in this model, which was extremely worse than that of the ulcer model of delayed skin wound healing (about 40%). Further, the ability to form granulation tissue was also poor (Fig. 4). From the above results, this model was considered to be a pathological model of intractable skin ulcer caused by diabetes.

### (4) Granulation tissue formation accelerating effect of bone marrow transplantation in ulcer model of delayed skin wound healing and model of intractable skin ulcer

Next, in order to examine whether or not bone marrow cells were involved in acceleration of granulation tissue formation, by using C57BL/6 mice, three types of models, i.e., Group 1: a group in which a GFT-Tg bone marrow-transplanted mouse was subjected to dorsal skin excision and ethanol exposure; Group 2: a group in which on the following day of 5-FU administration (intraperitoneal administration of 250 µl of 5-FU at a concentration of 11.25 mg/ml), dorsal skin excision and ethanol exposure, and GFP-Tg bone marrow transplantation were simultaneously carried out; and Group 3: a group in which on the following day of 5-FU administration (the same as above), GFP-Tg bone marrow transplantation was carried out, and on the following day, after it was confirmed that fluorescent cells completely disappeared from the blood, dorsal skin excision and ethanol exposure were carried out, were produced. An ability to form granulation tissue was examined 7 days after carrying out the dorsal skin excision and ethanol exposure in all the groups. As a result, the tissue repair ability was favorable in all the groups, and there was no difference among the 3 groups. However, when observation under a fluorescence microscope was carried out, in Group 1, GFP-positive cells were distributed in the entire thickness from the scab to the necrotic focus and the granulation tissue. On the other hand, in Group 2, the fluorescent cells were absent in the scab tissue, and in Group 3, the fluorescent cells were absent in the scab and also the necrotic focus under the scab. However, in all the groups, the GFP-positive cells were distributed in the granulation tissue, and the wound healing accelerating effect of the bone marrow transplantation was confirmed. Therefore, it was found that the bone marrow cells play an important role in healing of skin wound.

### (5) Change in the blood level of endogenous (mouse) G-CSF by skin wound

Next, based on the notion that mobilization of bone marrow cells is necessary for granulation tissue formation in the skin wound healing process, the in vivo kinetics of endogenous G-CSF in wound healing was examined. After a C57BL/6 mouse was subjected to dorsal skin excision and ethanol exposure in accordance with the method for producing an ulcer model of delayed skin wound healing, the plasma level of G-CSF was measured by the ELISA method (R&D system) using plasma obtained by orbital blood collection. As a result, on day 1 after skin excision and ethanol exposure, the blood G-CSF level increased to 100 times or more grater than the level of a non-treated control group, and thereafter, the level gradually decreased on day 2, and day 4 (Fig. 5). That is, it was revealed that before bone marrow-derived repair cells are present in the wound area after skin excision and ethanol exposure, a rapid increase in the blood G-CSF level occurs. Therefore, it was contemplated that there is a series of healing process of tissue damage → an increase in G-CSF → mobilization of bone marrow cells → tissue repair by the bone marrow cells → granulation tissue formation, and it was considered that an increase in G-CSF in the blood after tissue damage is an extremely physiological, logical and essential biological response.

### (6) Biological kinetics of megakaryocyte which is one of the bone marrow-derived cells by administration of G-CSF having an action of mobilizing bone marrow-derived cells

In the previously published reports, it is known that by subcutaneous administration of G-CSF to a mouse, hematopoietic stem cells are mobilized into the blood from the bone marrow, and also megakaryocytic progenitor cells (CFU-MK) also increase in the blood. However, because of using a cultural method, the accurate information on the amount thereof in the organ, the degree of differentiation thereof and the like remains unknown. Therefore, by using C57BL/6 mice, the distribution of megakaryocytes in the bone marrow, spleen, blood and other organs was examined in a direct manner based on the activity of CD41 and AChE (acetylcholine esterase), which are cell markers of megakaryocytes. With regard to the megakaryocytes in the tissues of the bone marrow in the femur, spleen, liver and lung, after the AChE activity was enzyme histochemically stained, the morphometry was carried out, and the number per unit area and size frequency were calculated. The megakaryocyte number in the blood was expressed as a cell number obtained as follows: microbeads were attached to CD41-positive cells, the CD41-positive cells were isolated using an automated magnetic cell sorter, and cells excluding platelets were counted using an automated hematocytometer. Consequently, when G-CSF (human recombinant G-CSF, lenograstim, trade name available from Chugai Pharmaceutical Co. Ltd., hereinafter the same shall apply) was subcutaneously administered (lumbar subcutaneous injection, hereinafter the same shall apply) at a dose of 250 µg/kg once daily for 7 consecutive days, megakaryocytes in the bone marrow gradually decreased, and after 7 days, megakaryocytes decreased to one third the level of the control group (Fig. 6). In particular, smaller sized megakaryocytes with an AChE activity decreased significantly (Fig. 7: An AChE-positive region in the bone marrow and spleen was defined to be the size of megakaryocyte, which was divided in 100 µm² each, and expressed as the megakaryocyte number of each size per unit area).
On the other hand, when CD41-positive cells, which are bone marrow-derived cells, in the blood were examined, although a small amount of CD41-positive cells are present even in a normal condition, by subcutaneous administration of G-CSF at a dose of 250 µg/kg once daily for 7 consecutive days, the cell number after 7 days increased to about 5 times greater than the level before the administration (Fig. 8: After completion of the G-CSF administration in each group, the mice were anesthetized, the whole blood was collected by cardiac puncture, platelets were removed and an isolated white blood cell fraction was used. CD41-positive cells were reacted with CD41 antibody + microbeads, and then isolated using an automated magnetic cell sorter, and the cell number was expressed as the number of CD41-positive cells excluding platelets counted using a hematocytometer). Further, when G-CSF of different concentrations was administered, a concentration-dependent increase in the CD41-positive cell number in the spleen was shown. In this way, the AChE activity or infiltration of CD41-positive cells by G-CSF administration was observed much in the spleen, however, it was hardly observed in the liver or lung.
The half-life of G-CSF in vivo is estimated to be about 4 to 5 hours. When G-CSF is subcutaneously administered to a C57BL/6 mouse at a dose of 250 µg/kg once daily for 7 consecutive days, granulocytes such as neutrophils gradually increase in the bone marrow and are mobilized into the blood and reach the maximum level after 7 days. It is also known that G-CSF mobilizes hematopoietic stem cells into the blood, however, it is considered that a protease such as neutrophil elastase or matrix matalloproteinase (MMP) cleaves the adhesive portion between cells and stroma. That is, there is also a possibility that megakaryocyte release into the blood due to G-CSF is also a secondary cellular response due to an increase in neutrophils in the bone marrow. In view of this, in order to elucidate whether or not the mobilization of megakaryocytes from the bone marrow is a direct response, only a single subcutaneous administration of G-CSF of different concentrations to C57BL/6 mice was carried out, and then, the change in megakaryocytes in the spleen thereafter was examined. As a result, by only a single subcutaneous administration of G-CSF, a concentration-dependent appearance of megakaryocytes in the spleen was observed. Further, subcutaneous administration of G-CSF once daily for 7 consecutive days, or only a single subcutaneous administration thereof to a C57BL/6 mouse at a dose of 250 µg/kg was carried out, and changes in the number of megakaryocytes in the spleen with time were compared. As a result, while an exponential increase was seen in the case of continuous administration, a linear increase was seen in the case of a single administration (Fig. 9: the first administration in the continuous administration and the single administration were carried out on day 1 in the horizontal axis, and the results were measured on the following day, i.e., on day 2 in the horizontal axis and are shown in the drawing. With regard to the case of the continuous administration, the results of administration on days 3, 5 and 7 are shown on days 4, 6, and 8 in the horizontal axis, respectively. With regard to the case of the single administration, the results on days 3, 5 and 7 after administration are shown on days 4, 6, and 8 in the horizontal axis, respectively). From these results, it was considered that by G-CSF administration, megakaryocytes were promptly released from the bone marrow and engrafted in the spleen and matured there.

### (7) Skin wound repair accelerating effect of G-CSF in ulcer model of delayed skin wound healing

Then, the wound healing accelerating effect of G-CSF having an action of mobilizing bone marrow cells was examined. To an ulcer model of delayed skin wound healing produced by using a C57BL/6 mouse, G-CSF was subcutaneously administered at a dose of 250 µg/kg once daily for 7 consecutive days, and an ability to form granulation tissue in the wound area was compared with that of a control mouse administered with a physiological saline solution. As a result, in the G-CSF administration group, the granulation tissue was thick, the proliferation of spindle-shaped cells was high and also matrix was rich, therefore, the ability to form granulation tissue was apparently enhanced. Accordingly, it was found that by G-CSF having an ability to mobilize bone marrow cells, the conditions of delayed skin wound healing is improved.

### (8) Effect of G-CSF administration on granulation tissue formation and kinetics of CD41-positive cell in blood using ulcer model of delayed skin wound healing and model of intractable skin ulcer

In order to examine the relationship between the granulation tissue formation response and the number of CD41-positive cells in the blood in an ulcer model of delayed skin wound healing and a model of intractable skin ulcer, the platelet number, CD41-positive cell number, and the thickness of granulation tissue were measured. The test groups were as follows: 1) Control group (normal mouse: Control), 2) Group subjected to skin excision and ethanol exposure (SP), 3) Group subjected to subcutaneous administration of G-CSF at a dose of 250 µg/kg once daily for 7 consecutive days after skin excision and ethanol exposure (SP+G-CSF), 4) Group subjected to skin excision and ethanol exposure on the following day of intraperitoneal administration of 250 µl of 5-FU at a concentration of 11.25 mg/ml (SP+5-FU), and 5) Group subjected to skin excision and ethanol exposure on the following day of intraperitoneal administration of 250 µl of 5-FU at a concentration of 11.25 mg/ml, and thereafter subjected to subcutaneous administration of G-CSF at a dose of 250 µg/kg once daily for 7 consecutive days (SP+5-FU+G-CSF). The results are shown in Table 1.

**[Table 1]**

| Platelet number, CD41-positive cell number and thickness of granulation tissue | | | | |
|---|---|---|---|---|
| Group | n | Platelets (10⁴/µl) | CD41-positive cells (10²/ml) | Thickness of granulation tissue (µm) |
| Control | 2 | 56.8 ± 5.2 | 467.1 ± 164.3 | - |
| Skin-peeling (SP) | 2 | 124.7 ± 35.9 | 309.3 ± 201.3 | 478.0 ± 86.2 |
| SP + G-CSF | 2 | 41.6 ± 2.4 | 634.1 ± 33.0 | 687.0 ± 99.0 |
| SP + 5-FU | 2 | 30.3 ± 5.7 | 185.7 ± 6.7 | 130.1 ± 3.3 |
| SP + 5-FU + G-CSF | 2 | 32.8 ± 24.9 | 390.4 ± 287.4 | 543.8 ± 354.3 |

As is apparent from Table 1, the platelet number increased by the skin excision and ethanol exposure compared with the control group, however, in the group subjected to G-CSF administration as well as skin excision and ethanol exposure, the platelet number decreased compared with the control group. The CD41-positive cell number decreased by about 35% by the skin excision and ethanol exposure compared with the control group, however, in the group subjected to G-CSF administration as well as skin excision and ethanol exposure, the CD41-positive cell number increased to about 2 times greater than that of the group subjected to only skin excision and ethanol exposure. The thickness of granulation tissue formed was increased by G-CSF administration. In particular, when the SP+5-FU group which was a model of intractable skin ulcer compared with the SP+5-FU+G-CSF group, the granulation tissue formation was apparently improved by G-CSF administration. When the correlation between the thickness of granulation tissue and the CD41-positive cell number or platelet number was examined, a strong positive correlation between the thickness of granulation tissue and the CD41-positive cell number was seen, however, there was no correlation between the thickness of granulation tissue and the platelet number (Fig. 10). From the above results, it was confirmed that subcutaneous administration of G-CSF is effective in both the ulcer model of delayed skin wound healing and the model of intractable skin ulcer.

### (9) Kinetics analysis of CD41-positive cells after G-CSF administration in ulcer model of delayed skin wound healing

As shown previously in Fig. 8, the CD41-positive cells begin to increase in the blood 3 days after G-CSF administration. When a GFP-Tg bone marrow-transplanted C57BL/6 mouse was subjected to dorsal skin excision and ethanol exposure, infiltration of a small amount of GFP-positive bone marrow-derived cells was observed from the following day, and an apparent increase thereof is observed 3 days thereafter, which coincided with the stage of initiation of granulation tissue formation. A C57BL/6 mouse to which G-CSF was subcutaneously administered at a dose of 250 µg/kg once daily for 3 consecutive days was subjected to dorsal skin excision and ethanol exposure, and on the following day, the blood was collected, and then, the number of CD41-positive cells in the blood was measured (G-CSF + Skin Peel). In addition, the number of CD41-positive cells in the blood of a mouse without G-CSF administration (control), the number of CD41-positive cells in the blood of a mouse subjected to only G-CSF administration collected on the following day of the G-CSF administration (G-CSF), the number of CD41-positive cells in the blood of a mouse subjected to only dorsal skin excision and ethanol exposure collected on the following day of the skin excision and ethanol exposure (Skin Peel) were measured. As a result, although the CD41-positive cell number increased in the group subjected to only G-CSF administration, in the group subjected to dorsal skin excision and ethanol exposure after G-CSF administration, the CD41-positive cell number significantly decreased compared with the group subjected to only G-CSF administration (Fig. 11). In the granulation tissue formed after the GFP-Tg bone marrow-transplanted C57BL/6 mouse was subjected to dorsal skin excision and ethanol exposure, GFP-positive and multinucleated cells with large cytoplasm were observed, and these cells were CD41-positive. Therefore, they were confirmed to be megakaryocytes. That is, it was considered that when there is a wound, the megakaryocytes mobilized into the blood from the bone marrow by G-CSF administration are recruited and infiltrated into the wound. Further, it was considered that because the megakaryocytes in a wound area have a lot of growth factors, they become important accelerating factors for granulation tissue formation.

### (10) Frequency of G-CSF administration and effect on shrinkage of wound area

Next, the frequency of G-CSF administration and the effect on shrinkage of wound area were examined, and whether or not the wound healing accelerating effect depends on the administration frequency was studied. To an ulcer model of delayed skin wound healing produced by using a C57BL/6 mouse, G-CSF at a dose of 250 µg/kg was subcutaneously administered once (G1), once daily for 2 consecutive days (G2) or once daily for 4 consecutive days (G4), and a relative wound area after 7 days was examined. As a result, as the frequency of G-CSF administration was increased, the wound area shrank, and by the administration thereof once daily for 4 consecutive days, the wound area shrank by about 10% more than that of the control group (C) (Fig. 12). Further, G-CSF at a concentration of 50 µg/ml was subcutaneously administered for 2 consecutive days such that the total administration amount of G-CSF was 10 µg, 20 µg and 40 µg, and a relative wound area after 7 days was examined. As a result, compared with the control group, the wound area shrank and the healing was accelerated depending on the concentration of administered G-CSF (Fig. 13).

### (11) Wound healing effect of G-CSF external agent in ulcer model of delayed skin wound healing

G-CSF was dissolved in an atherocollagen solution (a 3 mg/ml hydrochloric acid solution, hereinafter the same shall apply) at a concentration of 5 µg/200 µl, and the resulting solution was applied to a wound area of an ulcer model of delayed skin wound healing produced by using a C57BL/6 mouse, and the solution was coagulated. Then, the wound area was measured right after skin excision and ethanol exposure and after 7 days. As a result, there was no difference in the relative wound area after 7 days between the untreated group (w/o C) and the atherocollagen solution applied group (C). However, a decrease in the relative wound area by about 10% was observed in the atherocollagen solution and G-CSF applied group (C+G) (Fig. 14). Thus, it was found that G-CSF is useful as an active ingredient of an external agent for treatment of skin ulcer.

### (12) Concentration dependency of therapeutic effect of G-CSF external agent on wound area

G-CSF was dissolved in an atherocollagen solution at a concentration of 10, 20 and 40 µg/200 µl, and each of the resulting solutions was applied to a wound area of an ulcer model of delayed skin wound healing produced by using a C57BL/6 mouse, and the solution was coagulated. Then, the wound area was measured right after skin excision and ethanol exposure and after 7 days. As a result, there was no difference in the relative wound area after 7 days between the atherocollagen solution applied group (SPC) and the control group (SP). However, in the atherocollagen solution and G-CSF applied group (SPC+G10, G20 and G40), the relative wound area was reduced in a concentration dependent manner until the G-CSF concentration was 20 µg/200 µl. However, in the case where the G-CSF concentration was 40 µg/200 µl, the effect on shrinkage of wound area was decreased (Fig. 15). Thus, it was found that G-CSF is effective as an external agent for treatment of skin ulcer, and the effect is dose dependent.

### (13) Relationship between the different treatment methods using G-CSF for site subjected to skin excision and ethanol exposure and the change in the serum G-CSF level

With the use of ulcer models of delayed skin wound healing produced by using C57BL/6 mice, a group in which 100 µl of an atherocollagen solution was applied to a skin wound area and 5 or 20 µg of G-CSF was subcutaneously administered (subcutaneous administration group: G5sc and G20sc), a group in which a solution obtained by dissolving 5 or 20 µg of G-CSF in 100 µl of an atherocollagen solution was applied to a wound area and coagulated (atherocollagen single application group: C+G5 and C+G20), a group in which a solution obtained by dissolving 5 or 20 µg of G-CSF in 100 µl of an alginic acid solution (a 30 mg/ml phosphate buffer solution, hereinafter the same shall apply) was applied to a wound area and further 100 µl of an atherocollagen solution was applied thereto and coagulated (alginic acid single application group: A+G5 and A+G20), and a group in which after 100 µl of an atherocollagen solution was applied to a wound area, a solution obtained by dissolving 5 or 20 µg of G-CSF in 100 µl of an alginic acid solution was applied thereto, and further 100 µl of an atherocollagen solution was applied thereto and coagulated (atherocollagen and alginic acid combined application group: C+A+G5 and C+A+G20) were prepared. Then, by using them, after 6 hours, 12 hours, 18 hours, 1 day and 2 days, orbital blood collection was carried out, and by using the obtained plasma, the degree of leakage of the administered G-CSF into the mouse plasma was determined by the ELISA method. As a result, in all the groups, G-CSF in the plasma reached the maximum level after 6 hours, and thereafter, the G-CSF level gradually decreased, and after 2 days, it reached a level that could not be detected in all the groups. When the degrees of leakage of G-CSF into the blood were compared with regard to various G-CSF administration methods, the degree of leakage of G-CSF into the blood was the largest in the subcutaneous administration group, however, in the atherocollagen and alginic acid combined application group, atherocollagen single application group, and alginic acid single application group, the degree thereof was small. Further, when a trend of decrease of G-CSF in the plasma was examined, while G-CSF rapidly decreased in the subcutaneous administration group, the decrease of G-CSF was slow in the atherocollagen and alginic acid combined application group and atherocollagen single application group (Fig. 16). From the above results, it was considered that a method of applying G-CSF using at least either of atherocollagen and alginic acid as a substrate allows sustained release of G-CSF and has an effect on improving the retention of G-CSF in tissue.

### (14) Effect of G-CSF, collagen and alginic acid on granulation tissue formation after skin excision and ethanol exposure

With the use of ulcer models of delayed skin wound healing produced by using C57BL/6 mice, a group in which 100 µl of an atherocollagen solution was applied to a wound area and 5 µg of G-CSF was subcutaneously administered (subcutaneous administration group: G5sc), a group in which a solution obtained by dissolving 5 µg of G-CSF in 100 µl of an atherocollagen solution was applied to a wound area and coagulated (atherocollagen single application group: C+G5), a group in which a solution obtained by dissolving 5 µg of G-CSF in 100 µl of an alginic acid solution was applied to a wound area and further 100 µl of an atherocollagen solution was applied thereto and coagulated (alginic acid single application group: A+G5), and a group in which after 100 µl of an atherocollagen solution was applied to a wound area, a solution obtained by dissolving 5 µg of G-CSF in 100 µl of an alginic acid solution was applied thereto, and further 100 µl of an atherocollagen solution was applied thereto and coagulated (atherocollagen and alginic acid combined application group: C+A+G5) were prepared. Then, by using them, an epithelial regeneration area in an area around the wound and a granulation tissue formation area in the center of the wound area after 7 days from the initiation of the study were histologically examined. As a result, in the subcutaneous administration group, infiltration of inflammatory cells composed mainly of neutrophils was evident in the area around the wound, and epithelial regeneration was not favorable. On the other hand, in the atherocollagen single application group, alginic acid single application group and atherocollagen and alginic acid combined application group, granulation tissue formation was favorable in any of the cases. In particular, in the atherocollagen and alginic acid combined application group, the cell arrangement by fibroblasts was extremely good. Also in the granulation tissue formation area in the center of the wound area, infiltration of inflammatory cells composed mainly of neutrophils was less and the granulation tissue formation was more favorable in the atherocollagen single application group, alginic acid single application group and atherocollagen and alginic acid combined application group compared with the subcutaneous administration group. Among them, the granulation tissue formation was particularly favorable in the atherocollagen and alginic acid combined application group (Fig. 17). From the above results, it was found that by applying G-CSF to a wound area as a mixture with a hydrophilic high molecular substance such as collagen or alginic acid, the leakage of G-CSF into the blood is reduced, and G-CSF is retained in the tissue in the wound area, whereby an excellent effect is exhibited.

### (15) Therapeutic effect of G-CSF external agent on skin ulcer using model of intractable diabetic skin ulcer

By using models of intractable diabetic skin ulcer produced by using C57BL/6 mice, the effect of local application (subcutaneous injection and external application) of G-CSF was examined as follows. In G-CSF application group, a solution obtained by dissolving 30 µg of G-CSF in 150 µl of an atherocollagen solution was applied to a wound area and coagulated. In G-CSF subcutaneous administration group, a solution obtained by dissolving G-CSF in a physiological saline solution at a concentration of 5 µg/100 µl was administered twice, i.e., in the morning and evening, for 3 days (the total administration amount of G-CSF was 30 µg). Incidentally, in G-CSF non-administration group and G-CSF subcutaneous administration group, only 150 µl of an atherocollagen solution was applied to a wound area and coagulated. As a result, there was no significant difference among the 3 groups after 7 days from the initiation of the study in terms of the red blood cell count, white blood cell count and platelet number. The relative wound area after 7 days was about 90% in all the three groups, which was apparently worse than that of the ulcer model of delayed skin wound healing using a normal mouse (50%), however, thickness of the granulation tissue in the G-CSF administration group increased to about twice thicker than that of the G-CSF non-administration group, which showed a significant granulation tissue formation accelerating effect (Fig. 18).

### (16) Regarding effect of GM-CSF external agent as Comparative example on wound healing

GM-CSF is known as a mobilization factor for bone marrow cell-derived granulocyte-macrophage. The wound healing accelerating effect of GM-CSF was examined by using ulcer models of delayed skin wound healing. As GM-CSF, a mouse-derived recombinant (R&D System) was used. A solution obtained by dissolving GM-CSF in an atherocollagen solution at a concentration of 20 µg/100 µl was applied to a wound area of an ulcer model of delayed skin wound healing produced by using a C57BL/6 mouse and coagulated, and examination was carried out in the healing process over 7 days (SPC+GMCSF). As a control, a group in which only an atherocollagen solution was applied and coagulated was used (SPC). As a result, there was no difference between the two groups in terms of the body weight, white blood cell count, red blood cell count and platelet number before initiation of the study. After 7 days from the initiation of the study, a 1 to 2 g decrease in the body weight was observed in all the mice due to the stress caused by skin excision and ethanol exposure, however, there was no difference between the two groups. There was no difference between the two groups in terms of the red blood cell count and platelet number, and the white blood cell count slightly decreased in the SPC+GMCSF group, however, there was no significant difference in the white blood cell count. During the 7-day study, the wound area had symptoms like edema and so-called "swelling" was noted in the SPC+GMCSF group compared with the SPC group. When the shrinkage of the wound area after 7 days was examined, the relative wound area in the SPC+GMCSF group was larger by about 15% than that of the SPC group, and the shrinking effect of natural healing on a wound area was inhibited, and the wound area was exacerbated. When the thickness of granulation tissue was examined, the thickness thereof in the SPC+GMCSF group was slightly thicker than that of the SPC group. However, histologically, while proliferation of blood vessels, infiltration of inflammatory cells and proliferation of spindle-shaped fibroblasts were notably observed in the granulation tissue in the SPC group, edema was noted in the SPC+GMCSF group (Fig. 19). From the above results, it was found that in the ulcer models of delayed skin wound healing, the GM-CSF external agent does not have a wound healing accelerating effect, and rather enhances edema due to inflammation, and has an adverse effect on wound healing. From this, it was considered that for the treatment of skin ulcer, G-CSF which is capable of mobilizing a plurality of bone marrow-derived cells is the most suitable as a bone marrow cell mobilizing factor or a wound healing accelerating factor.

### (17) CXCR4 expression in megakaryocytes in bone marrow

By using a GFP-Tg bone marrow-transplanted C57BL/6 mouse, CXCR4 expression in the bone marrow and spleen after subcutaneous administration of G-CSF at a dose of 250 µg/kg once daily for 7 consecutive days was immunohistochemically examined. As a result, in the bone marrow, CXCR4 expression was observed specifically in GFP-positive megakaryocytes and stromal cells compared with other cells.

### (18) SDF-1 expression in skin lesion area

SDF-1 expression is not observed at all in skin tissue of a normal area of a C57BL/6 mouse. By the dorsal skin excision and ethanol exposure performed in accordance with the production method for an ulcer model of delayed skin wound healing, the granulation tissue formation response in the surrounding tissue was evident in the epidermal margin. When the expression of SDF-1, which is a ligand for CXCR4, in regeneration site in the epidermal margin after 7 days from skin excision and ethanol exposure was immunohistochemically examined, SDF-1 was strongly expressed in epidermal cells. Further, the expression of SDF-1 was also observed in hair root cells around the marginal area.

### (19) Study of treatment of wound area by SDF-1

By using a mouse recombinant SDF-1α/CXCL12 (TECHNE Co, MN, USA), a treatment study was carried out. Only an atherocollagen solution or a solution obtained by dissolving SDF-1 in an atherocollagen solution at a concentration of 1 µg/200 µl was applied to a wound area of an ulcer model of delayed skin wound healing produced by using a C57BL/6 mouse and coagulated. The wound area was measured right after skin excision and ethanol exposure, and after 3 days and 7 days, and the relative wound area after 3 days and 7 days were examined. As a result, the atherocollagen solution and SDF-1 application group showed a tendency of decreasing the relative wound area after 3 days compared with the atherocollagen solution application group (C) (C: 77.1 ± 4.3%, SDF-1: 65.0 ± 8.4%; p = 0.06), and after 7 days, the atherocollagen solution and SDF-1 application group showed a significant decrease (C: 37.2 ± 3.3%, SDF-1: 29.4 ± 4.5%; p < 0.05) (Fig. 20). Accordingly, it was found that SDF-1 is useful as an active ingredient of the external agent for treatment of skin ulcer.

### (20) Effect of CD41-positive cells on proliferation of blood vessels and tissue

A device for evaluating new tissue generation was constructed by applying a cell-impermeable membrane with a pore size of 0.45 µm to the upper surface of a Millipore ring (outer diameter: 14 mm, inner diameter: 10 mm, thickness: 2 mm), placing a synthetic collagen sponge (microfibrillar collagen hydrochloride, NOVACOL, Bioplex Co., NJ, USA) with a thickness of about 2 mm and a weight of about 27 mg therein, and applying a cell-permeable polyethylene mesh membrane with a pore size of 100 µm to the bottom surface thereof (Fig. 21).
CD41-positive cells and CD41-negative cells were isolated and collected, respectively, from the bone marrow cells collected from GFP-Tg, using a magnetic cell sorter, and dispersed in a culture medium, and after the respective cell numbers were made equal (2 x 10⁶/200 µl = 1 x 10⁷/ml), each of the resulting culture media was injected into the synthetic collagen sponge. Further, a mixture obtained by mixing the CD41-positive cells and CD41-negative cells at the same ratio as that in the bone marrow (mixing the CD41-positive cells with the CD41-negative cells at a ratio of 3.75%) was also injected therein (BMC). Then, the Millipore ring was inserted into the skin at the dorsal area of a normal C57BL/6 mouse in such a manner that the cell-permeable membrane was brought into contact with the dorsal area of the mouse. 7 days after leaving it therein, the Millipore ring was taken out, and the degree of proliferation of tissue in the collagen matrix was examined.
As a result, the periphery of the synthetic collagen sponge showed red in color due to the newly generated tissue composed of immature capillary vessels including red blood cells and fibroblasts infiltrated into the collagen matrix. When morphometry of the red area on the synthetic collagen sponge was carried out, conversion of the collagen sponge into tissue was hardly observed in the cell-free group. On the other hand, in the CD41-positive cell group, conversion into tissue progressed about twice more than in the CD41-negative cell group and the BMC group (Fig. 22). Accordingly, it was found that the CD41-positive cell is useful as an active ingredient of the external agent for treatment of skin ulcer.
As in the above, although angiogenesis is an important process for granulation tissue formation, a technical method for objectively evaluating the degree of angiogenesis in vivo had not been known so far. However, with the use of the device for evaluating new tissue generation shown in Fig. 21, the degree of angiogenesis could be evaluated by monitoring whether or not angiogenesis occurs in the synthetic collagen sponge due to cell migration from the biological tissue. This device for evaluating new tissue generation can be used not only for evaluating a skin wound healing process, but also for evaluating a proliferation activity in various biological tissues. In this Example, the synthetic collagen sponge was used as an extracellular matrix sheet, however, the extracellular matrix sheet is not limited to a synthetic collagen sponge, and can be a sponge sheet or a gel sheet containing at least one or more extracellular matrix components such as elastin, fibronectin and laminin. Further, in the extracellular matrix sheet, a component which is capable of accelerating new tissue generation in the matrix components such as CD41-positive cells or a test component for examining whether or not new tissue generation in the matrix components can be regulated (accelerated or inhibited) can be incorporated. Examples of the cell-permeable membrane or cell-impermeable membrane include biomembranes, chemically modified biomembranes, synthetic membranes and the like. Such a membrane can be coated with an extracellular matrix component as described above.

| Formulation example 1: Lotion (1) | |
|---|---|
| | (g/100 ml) |
| Glycerin | 10 |
| Ethanol | 10 |
| 1,3-butylene glycol | 5 |
| Hydroxyethyl cellulose | 1 |
| Cetanol | 1 |
| SDF-1 | 0.0005 |
| Collagen | 0.3 |
| Citric acid monohydrate | 0.66 |
| Trisodium citrate dihydrate | 0.27 |
| Methyl parahydroxybenzoate | 0.1 |
| Purified water | 71.6695 |

A lotion for treatment of skin ulcer that has the above-mentioned composition and contains SDF-1 at a final concentration of 0.5 mg/100 ml (0.0005%) was prepared by a well-known manufacturing process for lotion. It is preferable to set the final concentration of SDF-1 to about 0.0005 to 1.5%, and that of collagen to about 0.1 to 3% appropriately.

| Formulation example 2: Lotion (2) | |
|---|---|
| | (g/100 ml) |
| Glycerin | 10 |
| Ethanol | 10 |
| 1,3-butylene glycol | 5 |
| Hydroxyethyl cellulose | 1 |
| Cetanol | 1 |
| G-CSF | 0.02 |
| Collagen | 0.3 |
| Sodium alginate | 5 |
| Citric acid monohydrate | 0.66 |
| Trisodium citrate dihydrate | 0.27 |
| Methyl parahydroxybenzoate | 0.1 |
| Sodium dihydrogen phosphate | 3 |
| Purified water | 63.65 |

A lotion for treatment of skin ulcer that has the above-mentioned composition and contains G-CSF at a final concentration of 20 mg/100 ml (0.02%) was prepared by a well-known manufacturing process for lotion. It is preferable to set the final concentration of G-CSF to about 0.002 to 1.5%, and that of alginic acid to about 0.1 to 10% appropriately.

| Formulation example 3: Lotion (3) | |
|---|---|
| | (g/100 ml) |
| Glycerin | 10 |
| Ethanol | 10 |
| 1,3-butylene glycol | 5 |
| Hydroxyethyl cellulose | 1 |
| Cetanol | 1 |
| SDF-1 | 0.0005 |
| G-CSF | 0.02 |
| Collagen | 0.3 |
| Sodium alginate | 5 |
| Citric acid monohydrate | 0.66 |
| Trisodium citrate dihydrate | 0.27 |
| Methyl parahydroxybenzoate | 0.1 |
| Sodium dihydrogen phosphate | 3 |
| Purified water | 63.6495 |

A lotion for treatment of skin ulcer that has the above-mentioned composition and contains SDF-1 and G-CSF at final concentrations of 0.5 mg/100 ml (0.0005%) and 20 mg/100 ml (0.02%), respectively, was prepared by a well-known manufacturing process for lotion.

### Formulation example 4: Lotion (4)

A lotion for treatment of skin ulcer was prepared in the same manner as in Formulation example 1 except that CD41-positive cells were used by dispersing them at a cell density of 1 x 10⁹ cells/100 ml instead of using SDF-1 in Formulation example 1.

| Formulation example 5: Cataplasm (1) | |
|---|---|
| | (g/100 ml) |
| Glycerin | 7 |
| 1,3-butylene glycol | 3 |
| Pentylene glycol | 5 |
| Phenoxyethanol | 0.5 |
| Methylparaben | 0.1 |
| G-CSF | 0.02 |
| Sodium alginate | 5 |
| Sodium dihydrogen phosphate | 3 |
| Purified water | 76.38 |

A cataplasm for treatment of skin ulcer was prepared by impregnating a medicinal concentrated solution that has the above-mentioned composition and contains G-CSF at a final concentration of 20 mg/100 ml (0.02%) into a non-woven cloth by a well-known manufacturing process for cataplasm.

| Formulation example 6: Cataplasm (2) | |
|---|---|
| | (g/100 ml) |
| Glycerin | 7 |
| 1,3-butylene glycol | 3 |
| Pentylene glycol | 5 |
| Phenoxyethanol | 0.5 |
| Methylparaben | 0.1 |
| G-CSF | 0.02 |
| Collagen | 0.3 |
| Sodium alginate | 5 |
| Sodium dihydrogen phosphate | 3 |
| Purified water | 76.08 |

A cataplasm for treatment of skin ulcer was prepared by impregnating a medicinal concentrated solution that has the above-mentioned composition and contains G-CSF at a final concentration of 20 mg/100 ml (0.02%) into a non-woven cloth by a well-known manufacturing process for cataplasm.

### Formulation example 7: Wound dressing

A wound dressing for treatment of skin ulcer was prepared by applying the medicinal concentrated solution of Formulation example 5 to a dressing material composed of polyurethane film for wound dressing.

### Industrial Applicability

The present invention has industrial applicability in the point that it can provide a novel external agent for treatment of skin ulcer which has an excellent healing effect on intractable skin ulcer such as bedsore, diabetic skin ulcer and ischemic skin ulcer.

## Claims

1. An external agent for treatment of skin ulcer, **characterized in that** it comprises a composition containing at least one selected from the group consisting of granulocyte colony stimulating factor (G-CSF), stromal cell-derived factor-1 (SDF-1) and CD41-positive cells, and a hydrophilic high molecular substance.

2. The external agent for treatment of skin ulcer according to claim 1, **characterized in that** the hydrophilic high molecular substance is at least one selected from the group consisting of collagen, alginic acid and salts thereof.

3. An agent for local application for treatment of intractable skin ulcer, **characterized in that** it contains G-CSF as an active ingredient.

4. An agent for treatment of skin ulcer, **characterized in that** it contains SDF-1 as an active ingredient.

5. An agent for treatment of skin ulcer, **characterized in that** it contains CD41-positive cells as an active ingredient.

6. An animal model of skin ulcer, **characterized in that** it is produced by administering an anti-tumor drug to a non-human animal and 1 to 3 days thereafter, excising the dorsal skin of the animal.

7. A device for evaluating new tissue generation, **characterized in that** it comprises an extracellular matrix sheet having a predetermined thickness, a cell-permeable membrane and a cell-impermeable membrane, in which the cell-permeable membrane is disposed on one face of the extracellular matrix sheet and the cell-impermeable membrane is disposed on the other face of the extracellular matrix sheet, and that a biological tissue is brought into contact with the cell-permeable membrane as the facing surface, and whether or not new tissue generation occurs in the matrix components by cell migration from the biological tissue is monitored, whereby the degree of new tissue generation can be evaluated.
